# EUROPEAN PATENT APPLICATION

(11) **EP 3 162 371 A1**
(43) Date of publication of application: **03.05.2017**
(21) Application number: 15003065.8
(22) Date of filing: 27.10.2015
(51) Int. Cl.: A61K 31/695, A61K 9/50, A61K 45/06, A61K 38/46, A61K 38/47, A61K 38/48, A61P 1/00, A61P 1/04

(54) **A COMPOSITION COMPRISING SIMETHICONE, GASTRIC ACID-NEUTRALIZING SUBSTANCES AND GASTRIC ENZYME(S) (E.G. PANCREATINE) FOR USE IN THE TREATMENT OF DIGESTIVE DISORDERS**

(71) Applicant: Taciak, Przemyslaw, 08-124 Mokobody (PL); Mazurek, Adam, 01-518 Warszawa (PL)
(72) Inventor: Taciak, Przemyslaw, 08-124 Mokobody (PL); Mazurek, Adam, 01-518 Warszawa (PL)
(74) Representative: Patpol Kancelaria Patentowa Sp. z o.o.

(57) **Abstract**

The invention relates to a product comprising an innovative combination of components of different mechanisms of action, administered for gastric disorders. Primary components of the product are gastric acid-neutralizing agents, pancreatic enzymes and simethicone. Activities of all primary components of the product are based on physical action inside the human body, with pharmacological action having a minor significance. Moreover, to provide efficient performance and release of the active principles, suitable product forms were developed. A pharmaceutical form comprises subunits. A pharmaceutical form provides isolation of the active substances which otherwise could lead to mutual incompatibilities or their reduced stability. The target form ensures also release of the particular active substances in their sites of activity.

## Description

The invention relates to a product comprising an innovative combination of components with different mechanisms of action, used for gastric disorders. Primary components of the product are gastric acid-neutralizing agents, pancreatic enzymes and simethicone. Activities of all primary components of the product are based on physical or chemical, rather than pharmacological action inside of the human body. Therefore, it is a product which combines high therapeutic efficiency with concomitant reduction to a minimum of the possible adverse effects. Medical articles, in the European Union countries as well as worldwide, due to consideration of safety of use while maintaining therapeutic efficacy are subject to simplified procedures of authorization for use compared with medicinal products. Moreover, to provide efficient performance and release of the active ingredients, suitable product forms have been developed. A pharmaceutical form comprises subunits. A pharmaceutical form also permits to construct the product in a dose adapted for individual need of therapy, including specific age groups, without interfering with integrity of the form, as it happens in the case of disturbing integrity of the form while dividing tablets to adjust dosages. A pharmaceutical form provides isolation of the active substances which otherwise could cause mutual incompatibilities or their reduced stability. The target form ensures also release of the particular active substances in their sites of activity.

### PRIOR ART

Simethicone is a surfactant agent, a mixture of polydimethylsiloxane with silica (silica-activated dimethicone). It is mainly used for the treatment of flatulence and dyspepsia. The mechanism of action of simethicone involves lowering surface tension of the thin mucous film in the intestine lumen, by agglutinating the molecules of the mucus with polydimethylsiloxane droplets leading to breaking the mucus layer surface and releasing the gas. Gases released in that manner could be absorbed by intestinal walls or removed from the gastrointestinal tract in consequence of the peristaltic movements. Simethicone is not absorbed from the gastrointestinal tract after oral administration, and it does not exhibit pharmacological activity. It is excreted in the unaltered form with faeces. Simethicone does not influence absorption of nutrients. It does not alter reaction and volume of the gastric juice. A substitute for simethicone is dimethicone, which was also used in the instant invention.

Pancreatine is acquired from the porcine pancreas. Pancreatine includes the following enzymes: lipase, amylase and proteases. Lipase hydrolyzes fats, amylase degrades starch, and proteases degrade proteins. Said enzymes provide their activities efficiently within the small intestine environment only. Therefore it is most important for the pharmaceutical form employed to convey the enzymes, with no risk of decomposition (e.g. in the acidic stomach environment), into the duodenum. The duodenum is a natural site for physiological activity of the enzymes comprised in pancreatine. The effect of pancreatine is analogous to activities of the endogenous pancreatic enzymes. This results in the improvement in digestion and more efficient assimilation of nutrients comprised in the ingested food. Pancreatine is used *inter alia* in fat, protein and carbohydrate digestion disorders, which are caused by insufficient exocrine pancreas activity, pancreatic duct or common bile duct obstruction. The above-described enzymes act only locally, on the basis of a chemical reaction by hydrolyzing the nutrients. Pancreatine does not exert any systemic effect and does not interact with the human body through a pharmacological mechanism.

Activity of the gastric acid-neutralizing substances is based on the chemical reaction of the acid - base type. The natural reaction of the stomach environment has the acidic character. In contrast, the pH of the gastric juice being too low is not a physiologically natural phenomenon and could be a source of gastrointestinal tract ailments and the feeling of discomfort. Untreated hyperacidity can lead to severe affections of the digestive system, for example the gastric ulcer. Agents for neutralizing this gastric acid excess assist in restoring the physiological state. They are also employed for the treatment of pyrosis, dyspepsia, as well as reflux (leaking of the stomach contents to the esophagus). A number of the gastric acid-neutralizing substances are known (and are listed later in the patent specification). All the compounds of the present invention act in the chemical way by neutralizing hydrochloric acid from the digestive system environment. However, a number of minor differences in activities of the particular compounds could be indicated, which lead to different indications of the active ingredients. For example, magnesium carbonate is predominantly used for the symptomatic treatment of disorders related to hyperacidity of the gastric juice. On the other hand, aluminium hydroxide finds further utility in esophagitis and other disorders. However, the mechanism of action is common in the treatment of these symptoms. Considering the above, a number of preparations were developed which contain as their ingredients various gastric acid-neutralizing compounds of potential use in various disorders of the digestive system.

All of the discussed substances act upon the human body through the physical or chemical mechanism. The preparation, for the use indicated in the present application, does not act in the pharmacological, metabolic or immunological way. Use of three different substances presenting such character of activity in the treatment of gastric disorders is an innovative solution. Moreover, the preparation acts within all segments of the gastrointestinal tract: stomach, duodenum, intestines. A synergistic therapeutic effect has been thus obtained, with the high safety profile of the preparation provided through the non-pharmacological action.

The activity site for the gastric acid-neutralizing agents is the gastric lumen. Pancreatine exhibit its activity primarily in the duodenum. On the other hand, the activity site for simethicone is mainly the small intestine. The developed preparation is characterized by an innovative combination of the discussed components. The product obtained hereby is characterized by the overall multi-mechanism activity. Owing to this, the synergistic effect is obtained in the treatment of the digestive system disorders connected with dyspepsia, pyrosis, overeating, ethyl alcohol intoxication, hypoactivity or defective functioning of the pancreas.

Due to the multi-mechanism activity of the product, it can be successfully employed in the case of the digestive system problems. It is an innovative solution comprising the combination of the active ingredients with different mechanisms of action. Thus, a patient will not be made to recognize a cause of the gastric - enteric disorders. It should be emphasized that all substances, both active and auxiliary ones, are safe in the use. By employing analyzed forms of the pharmaceutical preparations, no mutual incompatibilities are met in simultaneous use of several substances.

The composite preparation comprised of the above-disclosed active ingredients, finds its utility in the therapy of the generalized digestive disorders, fat, protein, carbohydrate digestion disorders, hyperacidity, pyrosis, reflux, flatulence, dyspepsia, postprandial discomfort feeling. Indications to use for the complex preparation are also indications for the products with an individual active ingredient that is present in the inventive preparation.

### EXPERIMENTAL PART

Pharmaceutical preparation forms were designed and analyzed in such a manner that each of the active ingredients was released in a site of the optimal activity thereof. The developed forms of pharmaceutical preparations ensure efficient action of all the components and hinder their decomposition or deactivation.

Due to the lack of stability in the lower pH environments and susceptibility to the digestion processes by gastric enzymes, the pancreatic enzymes were employed in the invention in subunits of the pharmaceutical form of: pellets, microtablets or capsules and microcapsules. Each of the subunits is characterized by having an individual coat to ensure the lack of the release of the active substances (pancreatic enzymes) in the stomach in fasting state and postprandially (pH = 1 - 5). The individual compositions of subunits (pharmaceutical forms) as such for the enzymes are not the subject of the present invention. Instead, the invention provides the use thereof in combination with two or more components administered for the gastric problems, and in a single pharmaceutical form comprising two or more components acting on the basis of the non-pharmacological interaction with the human body. We have employed here, inter alia, multicompartmental systems. Such a form ensures release of the pancreatic enzymes at the pH of the duodenum and small intestine and maintaining suitable quality and stability, and efficacy.

Since they are targeting the stomach, the neutralizing compounds must be released from the pharmaceutical form at the acidic pH. Because of their reactivity, that cannot get in direct contact with the "coatings" employed for formulating the pancreatic enzymes, since they could damage the coatings, which applies equally to pellets, as well as capsules / microcapsules / minicapsules, tablets / microtablets / minitablets, so the neutralizing compounds must be separated from them. To ensure such separation, the subunits were employed in the pharmaceutical form of: microcapsules or multicompartmental microcapsules, or microtablets uncoated or coated with a hydrophilic film. The preparation employed is directed to assure rapid release of the included components in the stomach's environment.

Simethicone is a fluid substance which is administered orally mainly in soft capsule, lozenge, and granule forms. In the instant invention, simethicone was enclosed in a subunit which could ensure the lack of any direct contact with the remaining active components.

The target pharmaceutical form is a composite capsule or multicompartmental form comprising at least three above-described active substances. The forms as above demonstrated the highest efficacy for the combination of the discussed components.

The multicompartmental system is an oral pharmaceutical form which comprises at least 2 subunits with at least 3 active substances. The subunits could be - suitably miniaturized - tablets, capsules or a granulate, powder, etc. It is possible to 'enclose' the above subunits in a capsule or to tablet them. A number of solutions is already available commercially. In the course of the preparative work connected with the application for the patent, experiments employing various methods were conducted. Two of the systems appeared the best: a form of large composite capsules filled with suitable subunits or a 2- or 3-compartment system (a capsule divided into 2 or 3 parts by a partition). The best solutions are presented below.

In the case of a multicompartmental form being a composite capsule: the capsule includes inside a subunit: a microcapsule with simethicone, a subunit: a microtablet with the neutralizing substances, and subunits: pellets with enzymes (one or more). The capsules decompose in the stomach after ingestion. The gastric acid and enzymes cause also decomposition of the microtablets and release of the neutralizing substances and capsules with simethicone, while pellets are coated with a hydrophilic film with an enteric coating beneath. The hydrophilic film dissolves in the stomach, while the enteric coating provides protection for the enzymes in the pellet. The released neutralizing substances reduce acidity in the stomach and that is why the additional hydrophilic film is employed in the intraintestinal pellet. Simethicone is also released in the stomach, which act physically in the stomach and in more distal parts of the digestive system. Digestive enzymes are released not before the environment with pH above 6,5, i.e. in the duodenum. Depending on the intended therapeutic needs the capsule was filled with a different amount of the given subunit.

A more complex system as the above was also used. The multicompartmental system is divided into three regions (compartments): one compartment includes subunits of pellets with the pancreatic enzymes, the second one includes the neutralizing substances (as a mixture, minitablets or granulate), and the 3rd compartment includes a mixture or granulate with simethicone. Physiological activity of such a pharmaceutical form is analogous as in the case of use of the capsules with subunits described hereinabove.

A two-compartment form was also developed within the invention. The concept of the two-compartment system was based on the sites of activity of the particular active ingredients. The gastric acid-neutralizing substances are included in one compartment (as a mixture, minitablets or granulate), which releases the active ingredients in the stomach. The pellets with the pancreatic enzymes and the capsules with simethicone (or the minitablets with dimethicone) are included in the other compartment, which reaches beyond the stomach and only then releases the active ingredients. Use of the discussed pharmaceutical form is convenient also for the four or more-component preparations, for example pancreatine with the gastric acid-neutralizing substances, simethicone and the medicinal carbon.

The pharmaceutical forms disclosed hereinabove in the instant application are considered exemplary. In the course of the research work other forms were also developed, but the ones discussed above have demonstrated the best properties in the practical analyses. Miniaturized forms of tablets and capsules were obtained by commonly known methods. Some subunits used in the course of the experiments are commercially available (i.a. the minicapsules with simethicone).

Due to activity of the preparation through the chemical and/or physical mechanism the preparation is qualified as a medical product. According to the definition, a medical product is an agent, preparation, device, whose principal intended activity in or on the human body is not achieved by use of pharmacological, immunological or metabolic agents, but the activity thereof could be supported by such agents.

Compositions of the inventive product could include preservatives, sweeteners, dyes and antioxidants in its formula in maximum amounts specified by the home standards, including the regulation of the Minister of Health dated 22 November 2010 on the accepted auxiliary substances.

Composition of the preparation, depending of the form thereof, comprises the following active components (in parentheses, the amount of the substance in 1 dose is indicated):
Simethicone (from 0 to 400 mg)
Pancreatine or isolated enzymes, in combinations or individually (from 0 to 100000 units)
Dimethicone (from 0 to 400 mg)
Medicinal carbon (from 0 to 2000 mg)
Magnesium carbonate (from 0 to 1000 mg)
Magnesium oxide (from 0 to 1000 mg)
Magnesium peroxide (from 0 to 1000 mg)
Magnesium hydroxide (from 0 to 1000 mg)
Magnesium silicate (from 0 to 1000 mg)
Aluminium hydroxide (from 0 to 1000 mg)
Algeldrate (from 0 to 1000 mg)
Aluminium phosphate (from 0 to 1000 mg)
Monobasic aluminium carbonate (from 0 to 1000 mg)
Aluminium acetoacetate (from 0 to 1000 mg)
Aloglutamol (from 0 to 1000 mg)
Aluminium glycinate (from 0 to 1000 mg)
Calcium carbonate (from 0 to 1000 mg)
Calcium silicate (from 0 to 1000 mg)
Mixed salts of aluminium, calcium, magnesium (e.g. aluminium magnesium carbonate, calcium magnesium phosphate) (from 0 to 1000 mg)
Magaldrate (from 0 to 1000 mg)
Almagate (from 0 to 1000 mg)
Hydrotalcite (from 0 to 1000 mg)
Almasilate (from 0 to 1000 mg)
Basic bismuth(III) citrate (from 0 to 1000 mg)
Basic bismuth(III) nitrate (from 0 to 1000 mg)
Alginic acid (from 0 to 1000 mg)

Auxiliary substances, which were analyzed on developing the product:
Lactose (anhydrous or monohydrate) (from 0 to 500 mg)
Microcrystalline cellulose (from 0 to 500 mg)
Maize starch (from 0 to 500 mg)
Sucrose (from 0 to 500 mg)
Sorbitol (from 0 to 500 mg)
Mannitol (from 0 to 500 mg)
Povidone (from 0 to 500 mg)
Talc (from 0 to 500 mg)
Gelatin (from 0 to 500 mg)
Stearic acid (from 0 to 500 mg)
Magnesium stearate (from 0 to 500 mg)
Calcium hydrogenphosphate (from 0 to 500 mg)
Calcium dihydrogenphosphate (from 0 to 500 mg)
Hydroxyethylcellulose (from 0 to 500 mg)
Hydroxypropylcellulose (from 0 to 500 mg)
Hypromellose (from 0 to 500 mg)
Sodium carboxymethylcellulose (from 0 to 500 mg)
Sodium croscarmellose (from 0 to 500 mg)
Sodium stearylfumarate (from 0 to 500 mg)
Sodium carboxymethylstarch (from 0 to 500 mg)
Colloidal silica (from 0 to 500 mg)
Sodium laurylsulfate (from 0 to 500 mg)
Polyethylene glycol 4000 (from 0 to 500 mg)
Polyethylene glycol 8000 (from 0 to 500 mg)
Shellac (from 0 to 500 mg)
Cetyl alcohol (from 0 to 500 mg)
Cellulose acetate (from 0 to 500 mg)
Methylcellulose (from 0 to 500 mg)
Ethylcellulose (from 0 to 500 mg)
Polyethylene glycol 6000 (from 0 to 500 mg)
Cellulose acetate phthalate (from 0 to 500 mg)
Acetylcellulose succinate (from 0 to 500 mg)
Hydroxypropylmethylcellulose phthalate (from 0 to 500 mg)
Carnauba wax (from 0 to 500 mg)
Beeswax (from 0 to 500 mg)
Propylene glycol (from 0 to 500 mg)
Polyethylene glycol (from 0 to 500 mg)
Metacrylic acid copolymers (from 0 to 500 mg)
Glycerol (from 0 to 500 mg)
Croscarmellose (from 0 to 500 mg)
Crospovidone (from 0 to 500 mg)
Iron oxides (from 0 to 500 mg)
Preservatives (e.g. sorbic acid, nipagins) (from 0 to 100 mg)
Fragrances (e.g. carvone, limonene) (from 0 to 100 mg)
Essential oils ( e.g. peppermint, parsley) (from 0 to 100 mg)

The following examples demonstrate the detailed composition of the invention:

### 1. Capsule

### Example I

Magnesium oxide (minitablets, total 300 mg)
Simethicone (microcapsules, total 120 mg)
Pancreatine (pellets, total 12000 units)
Gelatin (400 mg)
Glycerol (30 mg)
Crospovidone (50 mg)

### Example II

Monobasic aluminium carbonate (minitablets, 350 mg)
Simethicone (minicapsules, total 100 mg)
Pancreatine (pellets, total 8000 units)
Medicinal carbon (50 mg)
Hypromellose (290 mg)
Peppermint oil (10 mg)

### 2. Three-compartmental system

### Example I

Magnesium carbonate (granulate 500 mg)
Simethicone (minicapsules, total 80 mg)
Pancreatine (pellets, total 10000 units)
Gelatin (300 mg)
Sodium laurylsulfate (10 mg)
Glycerol (25 mg)

### Example II

Magnesium hydroxide (minitablets, 320 mg)
Simethicone (minicapsules, total 60 mg)
Pancreatine (pellets, total 8000 units)
Hypromellose (290 mg)

### 2. Two-compartmental system

### Example I

Aluminium hydroxide (tabletka 250 mg)
Simethicone (minicapsules, total 100 mg)
Pancreatine (pellets, total 16000 units)
Croscarmellose (150 mg)
Gelatin (100 mg)
Polyethylene glycol (50 mg)

### Example II

Magaldrate (minitablets, 400 mg)
Simethicone (minicapsules, total 100 mg)
Pancreatine (pellets, total 12000 units)
Hypromellose (290 mg)
Sorbic acid (10 mg)

## Claims

1. A composition comprising:
a. simethicone, gastric acid-neutralizing substances and gastric enzyme(s) as principal components,
b. auxiliary substances,
**characterized in** comprising:
a. simethicone in an amount of 0,001 % to 99% by weight
b. gastric acid-neutralizing substances in an amount of 0,001 % to 99% by weight
c. gastric enzyme(s) in an amount of 0,001 % to 99% by weight.

2. The pharmaceutical composition of claim 1 **characterized in that** the composition can be presented as tablets, lozenges, chewable tablets, sublingual tablets, buccal tablets, coated tablets, modified-release tablets, capsules, soft capsules, modified-release capsules, multicompartmental systems (including capsules filled with minitablets, minicapsules, pellets, and granules; 2- and 3-compartmental capsules), syrup, suspension, powder for reconstitution into a suspension, solution, gel.

3. The pharmaceutical composition of claim 1 for use the treatment and/or prophylaxis and/or soothing of digestive ailments, failure of the digestive system, failure of secretory glands, dyspeptic conditions, gastroesophageal reflux, pyrosis, dyspepsia, excessive intestinal flatulence.

4. The pharmaceutical composition of claim 1 **characterized in that** the composition can be a medical product, drug product, dietary supplement, special nutritional medium.
